(19) Europäisches Patentamt
European Patent Office
Office européen des brevets

(11) Veröffentlichungsnummer : **0 368 211 B1**

# (12) EUROPÄISCHE PATENTSCHRIFT

(45) Veröffentlichungstag der Patentschrift :
**19.08.92 Patentblatt 92/34**

(51) Int. Cl.$^5$ : **C25B 3/10,** // C07C49/17

(21) Anmeldenummer : **89120519.7**

(22) Anmeldetag : **06.11.89**

## (54) Verfahren zur Herstellung von Dihydroxydionen.

(30) Priorität : **09.11.88 DE 3837954**

(43) Veröffentlichungstag der Anmeldung :
**16.05.90 Patentblatt 90/20**

(45) Bekanntmachung des Hinweises auf die Patenterteilung :
**19.08.92 Patentblatt 92/34**

(84) Benannte Vertragsstaaten :
**BE CH DE ES FR GB IT LI NL**

(56) Entgegenhaltungen :
**EP-A- 0 145 239**
**DE-A- 3 018 844**

(73) Patentinhaber : **BASF Aktiengesellschaft**
**Carl-Bosch-Strasse 38**
**W-6700 Ludwigshafen (DE)**

(72) Erfinder : **Hermeling, Dieter, Dr.**
**Zum Ordenswald 73f**
**W-6730 Neustadt (DE)**
Erfinder : **Becker, Rainer, Dr.**
**Im Haseneck 22**
**W-6702 Bad Dürkheim (DE)**
Erfinder : **Dobler, Walter, Dr.**
**Feuerbachstrasse 17**
**W-6900 Heidelberg (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Diese Erfindung betrifft ein neues elektrochemisches Verfahren zur Herstellung von Dihydroxydionen, wie 3,4-Dihydroxyhexan-2,5-dion.

Es sind schon mehrere Verfahren zur Herstellung von 3,4-Dihydroxyhexan-2,5-dion vorgeschlagen worden. So wird nach den Angaben in J. Chem. Soc. Perkin Trans I, (1985) 795, 3,4-Dihydroxyhexan-2,5-dion in einer mehrstufigen Synthese erhalten. Aus J. Org. Chem. 43, 4245 (1978) und J. Org. Chem. 38, 123 (1973) ist bekannt, daß man diese Verbindung durch ein- oder mehrstufige Oxidation von 2,5-Dimethylfuran mit Kaliumchlorat oder Osmiumtetroxid erhält. Der Umstand, daß 2,5-Dimethylfuran teuer ist und die explosiven und toxischen Oxidationsmittel schwierig zu handhaben sind, steht einer wirtschaftlichen Anwendung entgegen. Schließlich ist auch die in J. Org. Chem. 38, 123 (1973) beschriebene Zinkreduktion von Methylglyoxal zu 3,4-Dihydroxyhexan-2,5-dion wegen der Problematik, die dabei anfallenden Zinkabfälle entsorgen zu müssen, unvorteilhaft.

Es war deshalb nach einem Verfahren zu suchen, das es erlaubt, Dihydroxydione, wie 3,4-Dihydroxyhexan-2,5-dion auf vorteilhaftere Weise herzustellen. Nach dem Verfahren dieser Erfindung, durch das diese Aufgabe gelöst wird, stellt man Dihydroxydione der allgemeinen Formel

R-CO-CH(OH)-CH(OH)-CO-R    I

in der R für einen Alkylrest steht, dadurch her, daß man einen Aldehyd der allgemeinen Formel

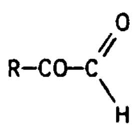

II,

in der R die obengenannte Bedeutung hat, in einem wasserhaltigen Elektrolyten, der einen pH-Wert kleiner als 7 aufweist, elektrolysiert.

Die Aldehyde der Formel II enthalten als Rest R einen Alkylrest mit z.B. 1 bis 6, vorzugsweise 1 bis 4 C-Atomen. Sie werden bei der erfindungsgemäßen Elektrolyse kathodisch dimerisiert. Dieses Ergebnis war nicht vorhersehbar, da aus Coll. Czech. Chem. 32, 1497-1504 (1967) bekannt ist, daß bei der Elektrolyse von Methylglyoxal bei pH-Werten über 7 durch kathodische Reduktion 1,2-Propandiol gebildet wird.

Das Verfahren der Erfindung kann sowohl in geteilten als auch in ungeteilten Elektrolysezellen durchgeführt werden, wobei man in geteilten Zellen bessere Ausbeuten erhält.

Als Anodenmaterialien werden z.B. Edelmetalle, wie Platin oder Metalloxide, wie $RuO_2$ eingesetzt. Bevorzugtes Anodenmaterial ist Graphit. Als Kathoden werden z.B. Eisen, Nickel oder Stahl, bevorzugt Graphit oder Blei verwendet.

Man elektrolysiert den Aldehyd der Formel II in einem Wasser enthaltenden Elektrolyten. Der Elektrolyt sollte außerdem ein Leitsalz enthalten, er kann auch einen aliphatischen Alkohol, wie Methanol oder Ethanol und als Säure Essigsäure enthalten. Als Leitsalze lassen sich alle üblicherweise als Leitsalze bekannten Salze einsetzen, die unter den Elektrolysebedingungen weitgehend stabil sind. Geeignete Leitsalze sind z.B. Sulfonate, z.B. die Alkalisalze von Benzolsulfonsäuren, wie $KSO_3Ph$ oder Acetate wie Kalium- oder Natriumacetat. Der Elektrolyt enthält beim Arbeiten mit geteilten Zellen zweckmäßigerweise Essigsäure, bei Verwendung von ungeteilten Zellen zweckmäßigerweise Natrium- oder Kaliumacetat.

Der Elektrolyt hat beispielsweise folgende Zusammensetung:

5 bis 40 Gew.% Aldehyd der Formel II
10 bis 90 Gew.% Wasser
0 bis 80 Gew.% Methanol oder Ethanol
0 bis 50 Gew.% Essigsäure
0,1 bis 5 Gew.% Leitsalz

Sein pH-Wert ist kleiner als 7 und liegt vorzugsweise bei 4 bis 6.

Die Stromdichten liegen bei dem erfindungsgemäßen Verfahren zwischen 0,5 und 25 $A/dm^2$, vorzugsweise bei 1 bis 5 $A/dm^2$. Man elektrolysiert bevorzugt drucklos und bei Temperaturen bis 100°C, zweckmäßigerweise bei 0 bis 90°C und vorzugsweise bei 40 bis 60°C. Die Elektrolyse kann sowohl diskontinuierlich als auch kontinuierlich durchgeführt werden.

Die Dihydroxydione kann man aus dem Elektrolyseaustrag auf an sich übliche Weise, z.B. durch Extraktion mit einem organischen Lösungsmittel gewinnen. Da die Löslichkeit des Dioldions in Wasser beträchtlich ist, ist eine kontinuierliche Extraktion, z.B. mit Essigsäureethylester vorteilhaft. Will man das nach dem Verfahren

der Erfindung erhaltene 3,4-Dihydroxyhexan-2,5-dion für die Herstellung des als Riechstoff bekannten 2,5-Di-methyl-4,5-dihydrofuranol-3-on-4 einsetzen, so kann man den Elektrolyseaustrag auch direkt zum gewünschten Endprodukt umsetzen. Entsprechende Cyclisierungsbedingungen werden z.B. in der DE-OS 2 845 843 beschrieben.

Beispiel

| | |
|---|---|
| Apparatur | : ungeteilte Zelle mit 11 Elektroden |
| Kathode | : Graphit |
| Elektrolyt | : 555 g Methylglyoxal (18,5 %) |
| | 940 g Wasser (31,3 %) |
| | 1496 g Methanol (49,9 %) |
| | 9 g Natriumacetat ( 0,3 %) |
| Anode | : Graphit |
| Stromdichte | : 3,3 $A/dm^2$ |
| Temperatur | : 45 bis 50°C |

Elektrolyse mit 1 F/mol Methylglyoxal. Der Elektrolyt wird während der Elektrolyse mit 200 l/h über einen Wärmeaustauscher gepumpt.

Nach beendeter Elektrolyse wird der Reaktionsaustrag am Rotationsverdampfer vom Lösungsmittel befreit. Es verbleiben 391 g Öl, welches nach gaschromatographischer Analyse 68 % 3,4-Dihydroxyhexan-2,5-dion enthält. Dies entspricht einer Ausbeute von 47 %, wobei 69 % als dl-Form und 31 % als meso-Form vorliegen. Dieses Gemisch kann direkt in die obengenannte Cyclisierungsreaktion zur Herstellung von 2,5-Di-methyl-4,5-dihydrofuran-3-ol-4-on eingesetzt oder aber einer kontinuierlichen Extraktion unterworfen werden. Durch 80-stündiges Extrahieren mit Essigester erhält man 234 g 3,4-Dihydroxyhexan-2,5-dion als Isomerengemisch, welches durch fraktionierte Kristallisation getrennt werden kann. Erhalten wird die dl-Form (FP: 89-91°C aus Essigester/Hexan) sowie die meso-Form (FP: 59-61°C aus Tetrachlorkohlenstoff/Chloroform).

## Patentansprüche

1. Verfahren zur Herstellung von Dihydroxydionen der allgemeinen Formel

R-CO-CH(OH)-CH(OH)-CO-R I

in der R für einen Alkylrest steht, dadurch gekennzeichnet, daß man einen Aldehyd der allgemeinen Formel

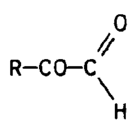 II,

in der R die obengenannte Bedeutung hat, in einem wasserhaltigen Elektrolyten, der einen pH-Wert von kleiner als 7 aufweist, elektrolysiert.

2. Verfahren nach Anspruch 1, dadurch gekennzeichnet, daß man die Elektrolyse in einer ungeteilten Durchflußzelle durchführt.

3. Verfahren nach Anspruch 1, dadurch gekennzeichnet, daß man die Elektrolyse in einer geteilten Zelle durchführt.

4. Verfahren nach Anspruch 1, dadurch gekennzeichnet, daß man bei Stromdichten von 0,5 bis 25 $A/dm^2$ und Temperaturen von 0 bis 90°C elektrolysiert.

5. Verfahren nach Anspruch 1, dadurch gekennzeichnet, daß man eine Kathode aus Graphit, Blei, Eisen, Nickel oder Stahl verwendet.

6. Verfahren nach Anspruch 1, dadurch gekennzeichnet, daß man für die Elektrolyse einen Elektrolyten mit der Zusammensetzung 5 bis 40 Gew.% Aldehyd der Formel II, 10 bis 90 Gew.% Wasser, 0 bis 80 Gew.% Methanol oder Ethanol, 0 bis 50 Gew.% Essigsäure und 0,1 bis 5 Gew.% eines Leitsalzes heranzieht.

## Claims

1. A process for the preparation of a dihydroxydione of the formula

R-CO-CH(OH)-CH(OH)-CO-R I

where R is alkyl, wherein an aldehyde of the formula

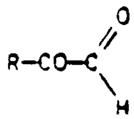

II

where R has the abovementioned meaning, is subjected to electrolysis in a water-containing electrolyte which has a pH of less than 7.

2. A process as claimed in claim 1, wherein the electrolysis is carried out in an undivided flow-through cell.

3. A process as claimed in claim 1, wherein the electrolysis is carried out in a divided cell.

4. A process as claimed in claim 1, wherein electrolysis is carried out at a current density of from 0.5 to 25 $A/dm^2$ and at from 0 to 90°C.

5. A process as claimed in claim 1, wherein a cathode of graphite, lead, iron, nickel or steel is used.

6. A process as claimed in claim 1, wherein an electrolyte composed of from 5 to 40% by weight of an aldehyde of the formula II, from 10 to 90% by weight of water, from 0 to 80% by weight of methanol or ethanol, from 0 to 50% by weight of acetic acid and from 0.1 to 5% by weight of a conductive salt is used for the electrolysis.

## Revendications

1. Procédé de préparation de dihydroxydiones de la formule générale

R-CO-CH(OH)-CH(OH)-CO-R I

dans laquelle R représente un radical alkyle, caractérisé en ce que l'on électrolyse un aldéhyde de la formule générale

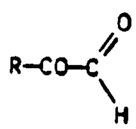

II,

dans laquelle R possède les significations qui lui ont été attribuées ci-dessus, dans un électrolyte contenant de l'eau, qui présente une valeur de pH inférieure à 7.

2. Procédé suivant la revendication 1, caractérisé en ce que l'on procède à l'électrolyse dans une cellule non divisée à passage direct.

3. Procédé suivant la revendication 1, caractérisé en ce que l'on procède à l'hydrolyse dans une cellule divisée.

4. Procédé suivant la revendication 1, caractérisé en ce que l'on procède à l'électrolyse à des densités de courant de 0,5 à 25 $A/dm^2$ et des températures de 0 à 90°C.

5. Procédé suivant la revendication 1, caractérisé en ce que l'on utilise une cathode en graphite, plomb, fer, nickel ou acier.

6. Procédé suivant la revendication 1, caractérisé en ce que l'on utilise, en vue de l'électrolyse, un électrolyte de la composition suivante : 5 à 40% en poids d'aldéhyde de la formule II, 10 à 90% en poids d'eau, 0 à 80% en poids de méthanol ou d'éthanol, 0 à 50% en poids d'acide acétique et 0,1 à 5% en poids d'un sel conducteur.